# EUROPÄISCHE PATENTSCHRIFT

(11) **EP 4 155 749 B1**
(45) Veröffentlichungstag und Bekanntmachung des Hinweises auf die Patenterteilung: **05.03.2025**
(21) Anmeldenummer: 21199517.0
(22) Anmeldetag: 28.09.2021
(51) Int. Cl.: G01R 33/36

(54) **MR-LOKALSPULE MIT DREHBAREM ANSCHLUSSKABEL**
MR LOCAL COIL WITH ROTATABLE CONNECTION CABLE
BOBINE LOCALE RM AVEC CÂBLE DE CONNEXION ROTATIF

(43) Veröffentlichungstag der Anmeldung: 29.03.2023
(73) Patentinhaber: Siemens Healthineers AG, 91301 Forchheim (DE)
(72) Erfinder: Driemel, Daniel, 09569 Oederan (DE); Hemmerlein, Martin, 96052 Bamberg (DE); Zink, Stephan, 90451 Nürnberg (DE)
(74) Vertreter: Siemens Healthineers Patent Attorneys

(56) Entgegenhaltungen:
- DE-A1- 102014 216 077
- DE-A1- 102015 217 325
- JP-A- 2008 154 933
- JP-A- H09 504
- US-A1- 2017 082 705

## Beschreibung

Die Erfindung betrifft eine MR-Lokalspule und eine Magnetresonanzvorrichtung.

In der Medizintechnik zeichnet sich die Bildgebung mittels Magnetresonanz (MR), auch Magnetresonanztomographie (MRT, engl. Magnetic Resonance Imaging, MRI) genannt, durch hohe Weichteilkontraste aus. Hierbei werden mit Hilfe einer Magnetresonanzvorrichtung Anregungspulse in ein Untersuchungsobjekt, welches in der Regel ein Patient ist, eingestrahlt. Dadurch werden im Patienten Magnetresonanzsignale ausgelöst. Die Magnetresonanzsignale werden als Messdaten von der Magnetresonanzvorrichtung empfangen und zur Rekonstruktion von Magnetresonanzabbildungen verwendet.

Oftmals erfolgt der Empfang der Magnetresonanzsignale mit so genannten Lokalspulen (engl. local coils), die oft auch Oberflächenspulen (engl. surface coils) genannt werden. Solche MR-Lokalspulen umfassen üblicherweise eine oder mehrere MR-Antennen und werden während einer Magnetresonanzuntersuchung in unmittelbarer Nähe des Patienten angebracht.

Eine konventionelle MR-Lokalspule weist zur Spannungsversorgung und Signalübertragung üblicherweise eine elektrische Verbindung zur Magnetresonanzvorrichtung auf. Dies wird üblicherweise durch ein Anschlusskabel realisiert. Das Anschlusskabel tritt aus der Lokalspule aus und wird mittels einer Steckverbindung mit der Magnetresonanzvorrichtung kontaktiert. Zur Untersuchung des Patienten werden Lokalspulen auch auf dem Patienten platziert. Sie bedecken oftmals einen Teil des Körpers des Patienten und sind nicht auf eine feste Position relativ zum Patienten, abhängig von der zu untersuchenden Region, beschränkt. Bei relativ zum Patienten flexibel positionierbaren MR-Lokalspulen ergibt sich je nachdem, wie die MR-Lokalspule auf dem Patienten angeordnet wird, eine unterschiedliche Führung des Anschlusskabels.

Die Druckschrift JP H09 504 A offenbart eine zweiteilige Lokalspule mit einem oberen und einem unteren Teil, die mittels eines Scharniers miteinander verbunden sind. Die Druckschrift US 2017/082705 A1 offenbart eine Lokalspule mit einem Anschlusskabel, das drehbar mit der Lokalspule verbunden ist. Die Druckschrift DE 10 2015 217325 A1 offenbart eine Lokalspulenanordnung, die direkt an einen Patiententisch angeordnet werden kann. Die Druckschrift JP 2008 154933 A offenbart eine Lokalspule mit einem Anschlusskabel zum Anschließen der Lokalspule an eine Magnetresonanzvorrichtung. Die Druckschrift DE 10 2014 216077 A1 offenbart eine Kopfspule, die mittels einer Direktsteckung kabellos mit einer Magnetresonanzvorrichtung verbunden werden kann.

Als Aufgabe der vorliegenden Erfindung kann insbesondere angesehen werden, die Handhabung einer MR-Lokalspule, insbesondere der Kabelführung, zu vereinfachen.

Die Aufgabe wird durch die Merkmale der unabhängigen Ansprüche gelöst. Vorteilhafte Ausgestaltungen sind in den abhängigen Ansprüchen beschrieben.

Demnach wird eine MR-Lokalspule vorgeschlagen, die eine Antenneneinheit mit zumindest einer Antenne zum Empfangen und/oder Senden von hochfrequenten Signalen, kurz: HF-Signalen, ein Anschlusskabel zum Anschließen der MR-Lokalspule an eine Magnetresonanzvorrichtung und ein flaches, insbesondere bandartiges, Übertragungselement zur Übertragung von, insbesondere elektrischer, Energie und/oder, insbesondere elektrischen und/oder optischen, Signalen zwischen dem Anschlusskabel und der Antenneneinheit umfasst. Dabei ist das Übertragungselement zumindest teilweise um eine Rotationsachse herum spiralförmig angeordnet, insbesondere aufgerollt. Erfindungsgemäß ist das Anschlusskabel ausgebildet, an einer Verbindungsstelle des Anschlusskabels zur Antenneneinheit um die Rotationsachse relativ zur Antenneneinheit gedreht zu werden.

Erfindungsgemäß beschreibt das Übertragungselement eine Spirale mit mindestens einer halben Windung, insbesondere mindestens einer Windung, insbesondere mindestens zwei Windungen um die Rotationsachse herum. Insbesondere beschreibt das Übertragungselement eine Schneckenkurve innerhalb einer Ebene oder Schicht. Insbesondere beschreibt das Übertragungselement keine Schraube, Wendel und/oder Helix.

Das Übertragungselement ist flach ausgestaltet. Insbesondere weist es eine geringe Dicke in Richtung zur Rotationsachse auf. Insbesondere weist das Übertragungselement eine Form eines Bandes auf. Vorzugsweise weist dieses Band eine Dicke auf, die mindestens drei, insbesondere fünf, mal geringer ist als die Breite des Bandes. Dadurch lässt sich das Übertragungselement leicht, insbesondere ohne oder nur geringem (mechanischen) Kraftaufwand, in ihre Spiralform bringen.

Die spiralförmige Anordnung des Übertragungselements beschreibt eine Kurve, insbesondere eine Schneckenlinie, die um die Rotationsachse verläuft und sich insbesondere entlang des Übertragungselements von der Rotationsachse (je nach Betrachterperspektive) entfernt oder sich ihr annähert. Je nach Rotationszustand kann die Außenlinie dieser Kurve einen kleineren oder größeren Abstand zur Rotationsachse aufweisen.

Die gesendeten HF-Signale sind beispielsweise Anregungssignale, insbesondere Anregungspulse, zur Anregung von Magnetresonanzsignalen. Die empfangenen HF-Signale sind beispielsweise Magnetresonanzsignale, die insbesondere in einem Körper eines Patienten mittels Magnetresonanz entstanden sind.

Die Magnetresonanzvorrichtung kann insbesondere ein Steckverbindungsteil, insbesondere eine Steckbuchse, umfassen, das mit einem komplementären Steckverbindungsteil des Anschlusskabels eine Steckverbindung eingehen kann. Über diese Steckverbindung können vorteilhafterweise die Signale und/oder Energie übertragen werden.

Das Anschlusskabel und/oder das Übertragungselement weisen vorzugsweise elektrische und/oder optische Leiter auf, um die Energie und/oder die Signale zu übertragen.

Vorzugsweise ist das Anschlusskabel lösbar an der (restlichen) MR-Lokalspule angeordnet, d.h. es kann entfernt werden, ohne die MR-Lokalspule zu beschädigen.

Vorzugsweise ist die Rotationsachse senkrecht zu einer, insbesondere flächigen, Oberfläche der Antenneneinheit angeordnet. Vorzugsweise weist diese Oberfläche eine gegenüber etwaigen anderen Oberflächen der MR-Lokalspule große Ausdehnung aus. Erfindungsgemäß kann das Anschlusskabel an der Verbindungsstelle zur Antenneneinheit gedreht werden.

Die Antenneneinheit frei platzierbarer MR-Lokalspulen (wie z.B. eine sogenannten Body-Arrays) können auf dem Patienten in unterschiedlichen relativen Lagen, beispielsweise um 0° oder 90° gedreht, zum Einsatz kommen. Somit verändert eine solche MR-Lokalspule im Gegensatz zum Beispiel zu einer MR-Kopfspule, die üblicherweise nicht frei platzierbar ist, oft ihre Lage und Entfernung zu ihrer Steckbuchse an der Magnetresonanzvorrichtung. (Die Steckbuchse kann beispielsweise am Patiententisch der Magnetresonanzvorrichtung angeordnet sein.) Durch die nun mögliche Drehung des Anschlusskabels kann die Länge des Anschlusskabels besonders kurz gehalten werden, wodurch vorteilhafterweise auch eine Reduzierung des Gewichts der MR-Lokalspule erreicht werden kann. Auch kann die Sicherheit des Patienten erhöht werden, wenn durch eine Vermeidung eines überlangen Kabels eine Schlaufenbildung auf dem Patienten vermieden werden kann.

Ferner ermöglicht die Drehbarkeit eine erhöhte Flexibilität bei der Handhabung der MR-Lokalspule, insbesondere bei ihrer Platzierung auf dem Körper des Patienten. Vorteilhafterweise kann die Führung des Anschlusskabels, insbesondere am Körper des Patienten, patientenfreundlicher gestaltet werden.

Eine weitere Ausführungsform der MR-Lokalspule sieht vor, dass das Übertragungselement eine flexible Leiterplatte umfasst. Die flexible Leiterplatte kann insbesondere ein- oder mehrlagig sein. Vorzugsweise kann sie eine impedanzkontrollierte Lage, insbesondere eine 50-Ohm-Lage, umfassen. Die flexible Leiterplatte weist vorzugsweise eine Dicke von 0,1 bis 2 mm, insbesondere 0,2 bis 0,8 mm, insbesondere 0,4 mm, auf. Die Normale der (flächigen) Oberflächen der Leiterplatte ist vorzugsweise (im Wesentlichen) senkrecht zur Rotationsachse ausgerichtet.

Durch die geringe Dicke der Leiterplatte und/oder ihren flexiblen Materialeigenschaften lässt sich die flexible Leiterplatte vorteilhafterweise leicht um die Rotationsachse wickeln. Auf der flexible Leiterplatte sind vorzugsweise elektrische Leiterbahnen, z.B. aus Kupfer, aufgebracht, über die Signale und/oder Energie übertragen werden können.

Eine weitere Ausführungsform der MR-Lokalspule sieht vor, dass das Übertragungselement ein Flachbandkabel umfasst. Das Flachbandkabel ist vorzugsweise ein mehradriges Kabel, in dem die Adern parallel nebeneinander geführt sind. Über die Adern können Signale und/oder Energie übertragen werden. Vorteilhafterweise kann das Flachbandkabel leicht um die Rotationsachse spiralförmig aufgewickelt werden.

Eine weitere Ausführungsform der MR-Lokalspule sieht vor, dass die Antenneneinheit mehrere Antennen, insbesondere Spulenelemente, umfasst, wobei das Übertragungselement mehrere nebeneinander angeordnete elektrische und/oder optische Leiter zur Übertragung der Signale der mehreren Antennen umfasst. Diese Leiter können insbesondere als Leiterbahnen auf einer flexiblen Leiterplatte und/oder als Adern in einem Flachbandkabel ausgeführt sein.

Jede der mehreren Antennen kann insbesondere einem Kanal zugeordnet sein, insbesondere eine Sendekanal und/oder einem Empfangskanal. Umfasst die Antenneneinheit beispielsweise 16 Antennen, kann insbesondere von einer 16-kanaligen MR-Lokalspule gesprochen werden.

Das Übertragungselement kann neben den Leitern, die einem bestimmen Kanal zugeordnet sind, auch weitere Leiter umfassen, mittels denen beispielsweise Steuerinformationen, Spulenidentifikationsdaten und/oder elektrische Leistung übertragen werden können.

Eine weitere Ausführungsform der MR-Lokalspule sieht vor, dass die Antenneneinheit insbesondere flächig und/oder flexibel, insbesondere in Form einer Decke, ausgebildet ist. MR-Lokalspulen mit einer derartigen Antenneneinheit kommt die Drehbarkeit des Anschlusskabels besondere zugute, da diese oftmals in sehr unterschiedlichen Orientierungen auf dem Patienten platziert werden. Vorteilhafterweise entfällt durch die vorgeschlagene Anbindung des Anschlusskabels an die Antenneneinheit die Notwendigkeit eine überschüssige Anschlussleitung verstauen zu müssen, und die Anschlussleitung verformt die flexible Antenneneinheit nicht am Spulenanschluss durch eine nicht optimale Lage.

Eine weitere Ausführungsform der MR-Lokalspule sieht vor, dass die MR-Lokalspule ein Gehäuse mit einem ersten Gehäuseteil und einem zweiten Gehäuseteil umfasst, wobei das Übertragungselement in dem Gehäuse angeordnet ist, wobei das zweite Gehäuseteil relativ zum ersten Gehäuseteil um die Rotationsachse rotierbar ist.

Vorzugsweise sind das erste und das zweite Gehäuseteil rigide. Vorteilhafterweise schützt das Gehäuse das Übertragungselement vor äußerer Einwirkung.

Vorzugsweise ist das zweite Gehäuseteil fest mit der Antenneneinheit verbunden. Insbesondere kann das erste Gehäuseteil wiederum mehrere Teile umfassen, und die Antenneneinheit kann zumindest eine Decklage umfassen, die zwischen besagten mehreren Teilen des ersten Gehäuseteils eingeklemmt ist.

Eine weitere Ausführungsform der MR-Lokalspule sieht vor, dass das Gehäuse eine Nut-Feder-Verbindung aufweist, wobei das zweite Gehäuseteil relativ zum ersten Gehäuseteil mittels der Nut-Feder-Verbindung geführt um die Rotationsachse rotierbar ist.

Vorzugsweise ist die Nut-Feder-Verbindung kreisförmig ausgeführt, wobei durch den Mittelpunkt des Kreises die Rotationsachse verläuft. Beispielsweise weist das erste Gehäuseteil einen Vorsprung auf, der in eine (um den Kreis) umlaufende Vertiefung (Nut) des zweiten Gehäuseteils eingreift. Dadurch kann das zweite Gehäuseteil relativ zum ersten Gehäuseteil während einer Rotation um die Rotationsachse stabil geführt werden.

Eine weitere Ausführungsform der MR-Lokalspule sieht vor, dass das Gehäuse einen Anschlag aufweist, der einen möglichen Rotationbereich um die Rotationsachse begrenzt. Vorteilhafterweise kann dadurch eine Überlastung des Übertragungselements durch eine zu starke Drehung vermieden werden.

Eine weitere Ausführungsform der MR-Lokalspule sieht vor, dass der zweite Gehäuseteil zwei Teile umfasst, die mittels Schrauben und/oder Rasten miteinander verbunden sind. Dies ermöglicht vorteilhafterweise eine einfache Fertigung und/oder Montage des zweiten Gehäuseteils.

Eine weitere Ausführungsform der MR-Lokalspule sieht vor, dass die MR-Lokalspule eine Starr-Flex-Leiterplatte umfasst, wobei das Übertragungselement ein flexibler Teil der Starr-Flex-Leiterplatte ist, wobei die Starr-Flex-Leiterplatte einen ersten starren Teil umfasst, der mittels einer ersten Verbindung mit dem ersten Gehäuseteil verbunden ist, wobei die Starr-Flex-Leiterplatte einen zweiten starren Teil umfasst, der mittels einer zweiten Verbindung mit dem zweiten Gehäuseteil verbunden ist.

Vorteilhafterweise ist die Starr-Flex-Leiterplatte so ausgelegt, dass eine Signalübertragung auch bei vielen Drehzyklen gesichert ist. Insbesondere ist die Starr-Flex-Leiterplatte platzsparender ausgeführt als eine etwaige Kabel-Litzen-Lösung. Vorteilhafterweise können auf der Starr-Flex-Leiterplatte neben der Signal- und/oder Energieführung noch andere Funktionen integriert werden (Lagesensor, Verstärker, Splitter, usw.).

Insbesondere weist der flexible Teil die Form eines Bandes auf, das spiralförmig um die Rotationsachse angeordnet ist. An einem ersten Ende des Bandes ist das erste starre Teil der Starr-Flex-Leiterplatte angeordnet, und an dem anderen Ende, dem zweiten Ende, des Bandes ist der zweite starre Teil der Starr-Flex-Leiterplatte angeordnet.

Die erste Verbindung und/oder die zweite Verbindung können insbesondere fest sein, d.h. der erste und/oder der zweite starre Teil der Starr-Flex-Leiterplatte können fest mit dem ersten bzw. zweiten Gehäuseteil verbunden sein.

Eine weitere Ausführungsform der MR-Lokalspule sieht vor, dass die erste Verbindung und/oder die zweite Verbindung lösbar ausgebildet sind, z.B. mittels einer Zwischensteckverbindung. Vorteilhafterweise ermöglicht dies eine leichtere Reparatur der MR-Lokalspule. Beispielsweise kann mit einer Zwischensteckverbindung an dem zweiten Gehäuseteil das Anschlusskabel leichter ausgetauscht werden. Vorteilhafterweise ist die Starr-Flex-Leiterplatte leicht mit einer Zwischensteckverbindung auszustatten und ermöglicht einen schnellen und sicheren Tausch der Anschlussleitung, beispielsweise bei der Montage oder einer möglichen Reparatur.

Eine weitere Ausführungsform der MR-Lokalspule sieht vor, das Übertragungselement ein erstes Ende und ein zweites Ende aufweist, wobei das Übertragungselement am ersten Ende und/oder am zweiten Ende, insbesondere um 90°, abgewinkelt ist. Insbesondere weist der flexible Teil der Starr-Flex-Leiterplatte ein erstes Ende und ein zweites Ende auf, wobei das Übertragungselement am ersten Ende und am zweiten Ende, insbesondere um 90°, abgewinkelt ist.

Vorteilhafterweise kann dadurch das Übertragungselement aus einer Schicht und/oder Ebene der spiralförmigen Anordnung herausgeführt werden. Dies ermöglicht beispielsweise eine leichtere Verbindung mit dem Anschlusskabel und/oder der Antenneneinheit.

Eine weitere Ausführungsform der MR-Lokalspule sieht vor, dass das erste Gehäuseteil und/oder das zweite Gehäuseteil einen Spalt umfasst, der senkrecht zur Rotationsachse verläuft, wobei das Übertragungselement durch den Spalt durchgeführt ist.

Insbesondere kann das abgewinkelte erste und/oder zweite Ende des Übertragungselements durch den Schlitz durchgeführt sein. Vorteilhafterweise dient der Spalt als Mitnehmer des um eine Rotationsachse herum spiralförmig angeordneten Teils des Übertragungselements. Insbesondere spannt bzw. entspannt sich dadurch die Spirale bei Rotation des Anschlusskabels relativ zur Antenneneinheit.

Eine weitere Ausführungsform der MR-Lokalspule sieht vor, dass das Gehäuse ein Führungselement umfasst, wobei zumindest der um die Rotationsachse herum spiralförmig angeordnete Teil des Übertragungselement in dem Führungselement angeordnet ist und von diesem geführt wird.

Ferner wird eine Magnetresonanzvorrichtung mit zumindest einer vorangehend beschriebenen MR-Lokalspule vorgeschlagen.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
Fig. 1 eine Magnetresonanzvorrichtung mit einer MR-Lokalspule in einer schematischen Darstellung,
Fig. 2-3 eine Anordnung mit einem Übertragungselement zur Übertragung von Energie und/oder Signalen zwischen einem Anschlusskabel und einer Antenneneinheit der MR-Lokalspule,
Fig. 4 ein Übertragungselement in einem ausgerollten Zustand,
Fig. 5 ein Übertragungselement in Spiralform in einem ersten Rotationszustand,
Fig. 6 ein Übertragungselement in Spiralform einem zweiten Rotationszustand,
Fig. 7 ein Element der Anordnung mit einem Spalt, in dem das Übertragungselement durchgeführt wird,
Fig. 8 ein Zwischengehäuse zur Aufnahme des Übertragungselements.

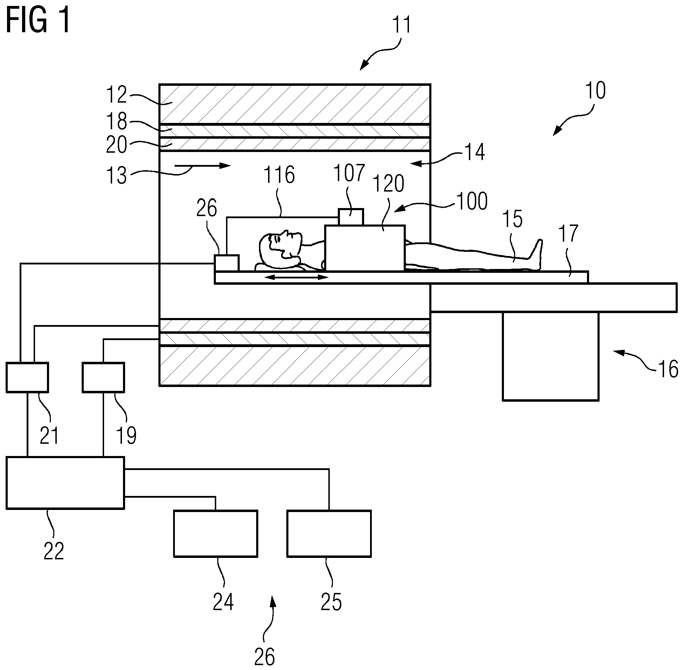

In Fig. 1 ist eine Magnetresonanzvorrichtung 10 mit einer MR-Lokalspule 100, die an einem Patienten 15 angeordnet ist, schematisch dargestellt. Die Magnetresonanzvorrichtung 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzvorrichtung 10 einen Patientenaufnahmebereich 14 zu einer Aufnahme des Patienten 15. Der Patientenaufnahmebereich 14 im vorliegenden Ausführungsbeispiel ist zylinderförmig ausgebildet und in einer Umfangsrichtung von der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Magneteinheit 11 umfasst weiterhin eine Hochfrequenzantenneneinheit 20, welche im vorliegenden Ausführungsbeispiel als fest in die Magnetresonanzvorrichtung 10 integrierte Körperspule ausgebildet ist. Die Hochfrequenzantenneneinheit 20 wird von einer Hochfrequenzantennensteuereinheit 21 der Magnetresonanzvorrichtung 10 gesteuert und strahlt Magnetresonanzsequenzen in Form hochfrequenter (HF) Signale in einen Untersuchungsraum ein, der im Wesentlichen von einem Patientenaufnahmebereich 14 der Magnetresonanzvorrichtung 10 gebildet ist. Dadurch stellt sich dem von dem Hauptmagneten 12 erzeugten Hauptmagnetfeld 13 eine Anregung von Atomkernen ein. Durch Relaxation der angeregten Atomkerne werden Magnetresonanzsignale, bei denen es sich wiederum um HF-Signale handelt, erzeugt. Die Hochfrequenzantenneneinheit 20 ist zum Empfang der Magnetresonanzsignale ausgebildet. Ferner umfasst die MR-Lokalspule eine Antenneneinheit 120 mit Antennen, die ebenfalls ausgebildet sind, HF-Signale zu empfangen und/oder zu senden. Vorteilhafterweise ist die Antenneneinheit 120 flexibel, beispielsweise wie eine Decke, ausgebildet, so dass die Antennen besonders nahe am Körper des Patienten 15 angebracht werden können.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung der Magnetresonanzsignale, die während der Magnetresonanzuntersuchung erfasst werden. Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzabbildungen können auf einer Anzeigeeinheit 24, beispielsweise auf zumindest einem Monitor, der Benutzerschnittstelle 23 für ein medizinisches Bedienpersonal angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die MR-Lokalspule 100 ist über ein Anschlusskabel 116 mit einer auf dem Patiententisch 17 angeordneten Steckplatz der Magnetresonanzvorrichtung 10 verbunden, über den Signale und/oder Energie von und/oder zu der Hochfrequenzantennensteuereinheit 21 übermittelt werden können.

An der Anschlussstelle zwischen Anschlusskabel 116 und der Antenneneinheit 120 der MR-Lokalspule befindet sich ein flaches Übertragungselement 107 zur Übertragung von Energie und/oder Signalen zwischen dem Anschlusskabel 116 und der Antenneneinheit 120, welches in den folgenden Figuren im Detail dargestellt wird. Insbesondere wird dabei ein Ausführungsbeispiel gezeigt, wie eine mechanische und elektrische Verbindung vom Anschlusskabel zur Antenneneinheit 120 der MR-Lokalspule 100 drehbar gestaltet werden kann.

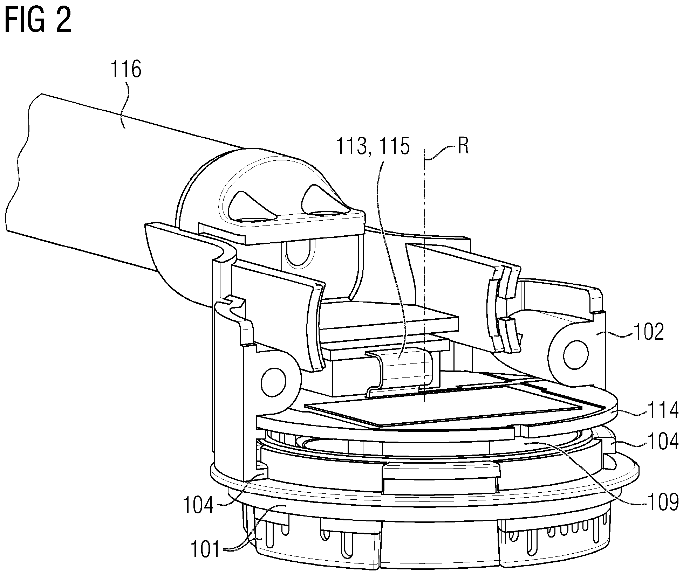

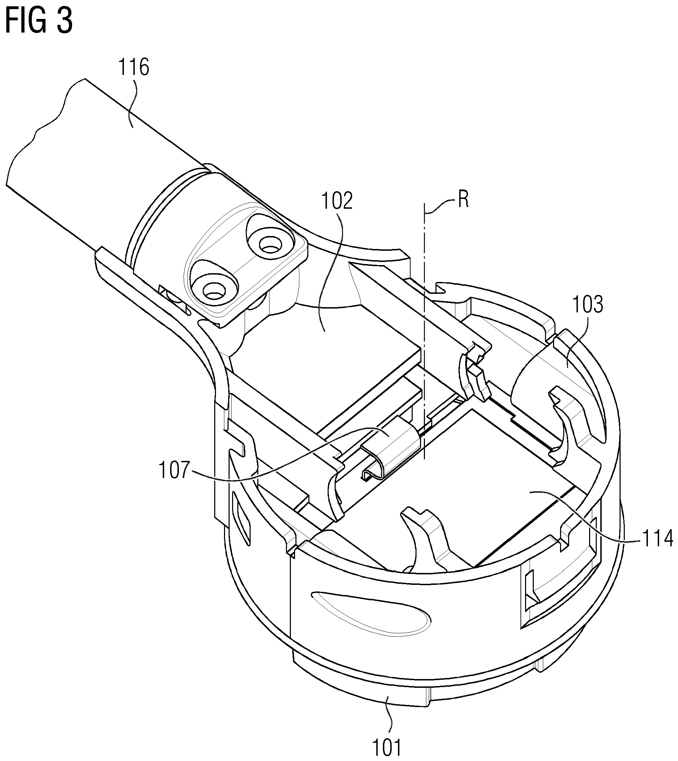

Wie in den Fig. 2 und 3 gezeigt wird, besteht die Anordnung aus mehreren Gehäuseteilen, die zueinander drehbar sind. Ein erstes Gehäuseteil 101 sind fest mit der Lokalspule verbunden. Dazu umfasst das erste Gehäuseteile beispielsweise zwei Teile, zwischen die eine Decklage der Antenneneinheit 120 eingeklemmt werden kann.

Ein weites Gehäuseteil umfassend zwei Teile 102, 103 ist drehbar zum ersten Gehäuseteil 101 gelagert. Das Drehgelenk wird über ein Nut-Feder-Verbindung 104 realisiert. Das erste Gehäuse ist zur besseren Montierbarkeit in zwei Teile 102 und 103 geteilt und wird zum Beispiel über Schrauben oder Rasten miteinander verbunden. Den Drehwinkel kann ein Anschlag zwischen den rotierenden Teilen, also dem ersten Gehäuseteil 101 und dem zweiten Gehäuseteil 102, 103 begrenzen.

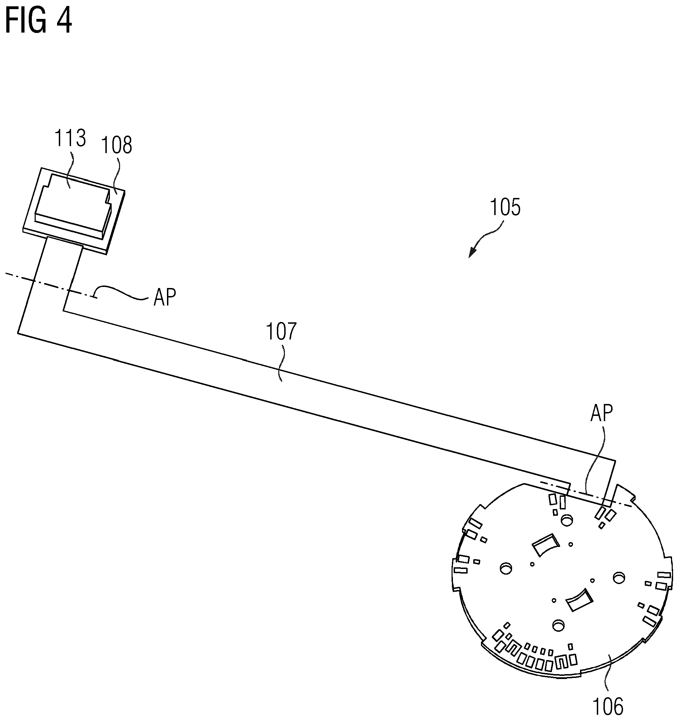

Die Kontaktierung erfolgt über eine Starr-Flex-Leiterplatte 105, die in Fig. 4 dargestellt ist. Die Starr-Flex-Leiterplatte 105 umfasst zwei starre Teile 106, 108 sowie einen flexiblen Teil in Form einer flexiblen Leiterplatte, die ein flaches Übertragungselement 107 bildet.

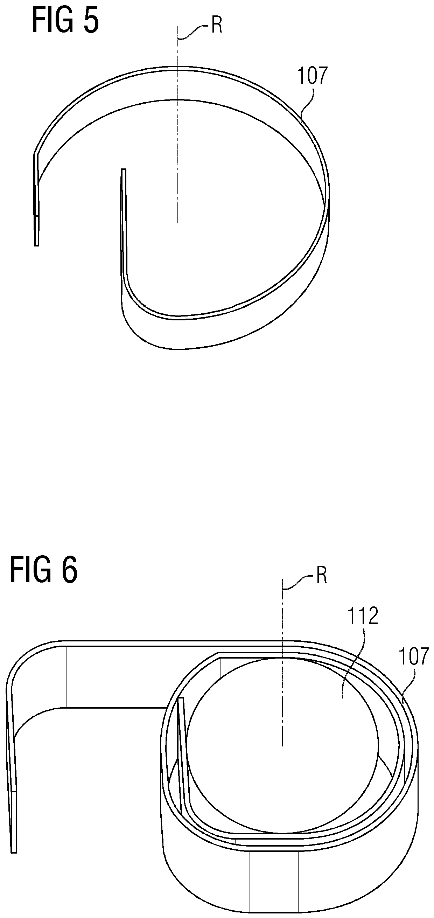

Der starre Teil 106 ist fest mit dem ersten Gehäuseteil 101 verbunden. Das Übertragungselement 107 bildet durch zweimaliges Abwinkeln an den Stellen AP und Aufrollen eine Spirale, die sich wie eine Spiralfeder durch die Rotation spannen, siehe Fig. 6, oder entspannen, siehe Fig. 5, kann. Das Übertragungselement 107 ist also teilweise um eine Rotationsachse R herum spiralförmig angeordnet.

Die Länge des Übertragungselements 107 und der minimale Wickeldurchmesser in Form eines Begrenzungselements 112, der verhindert, dass die Leiterbahnen des Übertragungselements 107 durch ständiges Spannen und Entspannen der Spirale brechen, definiert den erreichbaren Drehwinkel. Je länger der flexible Teil desto größer ist der erreichbare Drehwinkel des Anschlusskabels 116 zur Antenneneinheit 120 der MR-Lokalspule.

Am zweiten Gehäuseteil (102, 103) ist eine Leiterplatte 114 angeordnet, die zusammen mit dem zweiten Gehäuseteil (102, 103) gegenüber dem ersten Gehäuseteil (101) drehbar ist. Der starre Teil 108 der Starr-Flex-Leiterplatte 105 umfasst einen Stecker 113 (oder eine Buchse), der auf der Leiterplatte 114 in eine Buchse 115 (oder einen Stecker) gesteckt wird und somit eine lösbare Verbindung bildet. Diese Zwischensteckverbindung ermöglicht ein Austauschen des Anschlusskabels 116 der MR-Lokalspule 100. Es ist auch denkbar, auf diese Zwischensteckverbindung zu verzichten; dann entspricht der starre Teil 108 funktional der drehbaren Leiterplatte 114. An diese drehbare Leiterplatte 114 wird das Anschlusskabel 116, das die MR-Lokalspule 100 mit der Magnetresonanzvorrichtung 10 verbindet zum Beispiel mittels einer Lötverbindung angeschlossen. Die drehbare Leiterplatte 114 lässt eine filigranere Konfektionierung zu und ist über die Zwischensteckverbindung leicht bei der Endmontage der MR-Lokalspule 100 zu verbauen.

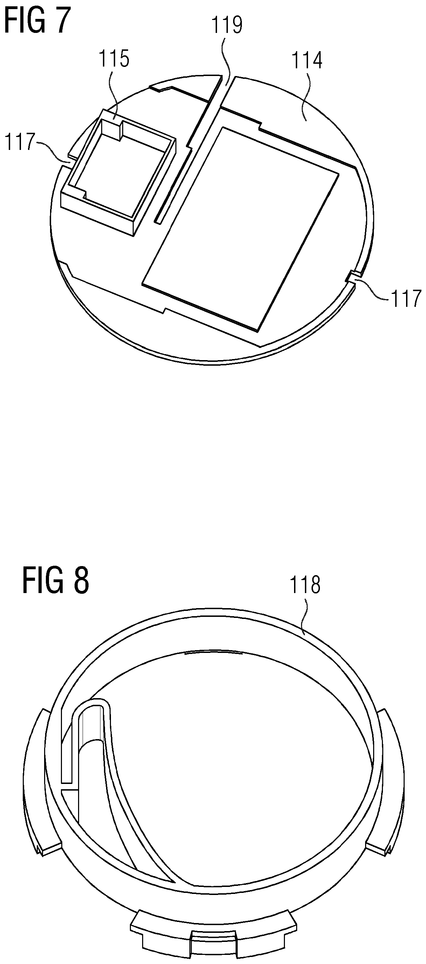

Wie in Fig. 7 zu sehen ist, weist die drehbare Leiterplatte 114 Aussparungen 117 auf, die mit Zapfen in den Teilen 102 und 103 des zweiten Gehäuseteils einen Formschluss bilden. Dieser Formschluss fungiert als Mitnehmer zwischen der Leiterplatte 114 und dem zweiten Gehäuseteil 102, 103. Die Leiterplatte 114 weist einen Spalt 119 auf. In diesen Spalt 119 wird das abgewinkelte Ende des Übertragungselement 107 mit dem starren Teil 108 eingeschoben und gelangt so zum Stecker 113 der Zwischensteckverbindung. Dieser Spalt 119 dient als Mitnehmer des spiralförmigen Übertragungselements 107, das sich somit bei Rotation der Leiterplatte 114 spannt und entspannt, siehe Fig. 5 und 6. Ein Zwischengehäuse, wie es in Fig. 8 dargestellt ist, dient als Führungselement 118, in dem sich das spiralförmige Übertragungselements 107 spannen und entspannen kann; außerdem dient es zu dessen Führung. Es ist fest mit dem ersten Gehäuseteil 101 verbunden.

Die Drehbarkeit bringt den Ausgang des Anschlusskabels 116 aus der MR-Lokalspule 100 idealerweise immer in die optimale Position zum Steckplatz 26 der Magnetresonanzvorrichtung. Das verbessert die Handhabung und optimiert die Lage des Anschlusskabels 116 auf dem Patienten 15. Durch das drehbar ausgeführte Anschlusskabel 116 kann die nötige Kabellänge verkürzt werden. Daraus folgt insbesondere, dass für das Anschlusskabels 116 weniger Mantelwellensperren benötigt werden. Dadurch erhöht sich die Flexibilität des Anschlusskabels 116 (der Bereich der Mantelwellensperre versteift des Anschlusskabels 116) und deren Gewicht sinkt.

Es wird abschließend noch einmal darauf hingewiesen, dass es sich bei der vorhergehend detailliert beschriebenen MR-Lokalspule und der Magnetresonanzvorrichtung lediglich um Ausführungsbeispiele handelt, welche vom Fachmann in verschiedenster Weise modifiziert werden können, ohne den Bereich der Erfindung zu verlassen, der durch die Ansprüche definiert wird. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. MR-Lokalspule umfassend
- eine Antenneneinheit (120) mit zumindest einer Antenne zum Empfangen und/oder Senden von HF-Signalen,
- ein Anschlusskabel (116) zum Anschließen der MR-Lokalspule (100) an eine Magnetresonanzvorrichtung (10),
- ein flaches Übertragungselement (107) zur Übertragung von Energie und/oder Signalen zwischen dem Anschlusskabel (116) und der Antenneneinheit (120),
wobei das Anschlusskabel ausgebildet ist, an einer Verbindungsstelle des Anschlusskabels zur Antenneneinheit um eine Rotationsachse relativ zur Antenneneinheit gedreht zu werden,
**dadurch gekennzeichnet, dass** das Übertragungselement (107) zumindest teilweise um die Rotationsachse (R) herum spiralförmig angeordnet ist, wobei das Übertragungselement eine Spirale mit mindestens einer halben Windung um die Rotationsachse herum beschreibt.

2. MR-Lokalspule nach Anspruch 1,
wobei das Übertragungselement (107) eine flexible Leiterplatte und/oder ein Flachbandkabel umfasst.

3. MR-Lokalspule nach einem der vorangehenden Ansprüche,
wobei die Antenneneinheit (120) mehrere Antennen umfasst,
wobei das Übertragungselement (107) mehrere nebeneinander angeordnete elektrische und/oder optische Leiter zur Übertragung der Signale der mehreren Antennen umfasst.

4. MR-Lokalspule nach einem der vorangehenden Ansprüche, wobei die Antenneneinheit (120) flächig ausgebildet ist.

5. MR-Lokalspule nach einem der vorangehenden Ansprüche, wobei die Antenneneinheit (120) flexibel ausgebildet ist.

6. MR-Lokalspule nach einem der vorangehenden Ansprüche, wobei die MR-Lokalspule (100) ein Gehäuse mit einem ersten Gehäuseteil (101) und einem zweiten Gehäuseteil (102, 103) umfasst,
wobei das Übertragungselement (107) in dem Gehäuse angeordnet ist,
wobei das zweite Gehäuseteil (102, 103) relativ zum ersten Gehäuseteil (101) um die Rotationsachse (R) rotierbar ist.

7. MR-Lokalspule nach Anspruch 6,
wobei das Gehäuse eine Nut-Feder-Verbindung (104) umfasst,
wobei das zweite Gehäuseteil (102, 103) relativ zum ersten Gehäuseteil (101) mittels der Nut-Feder-Verbindung (104) geführt um die Rotationsachse (R) rotierbar ist.

8. MR-Lokalspule nach einem der Ansprüche 6 oder 7,
wobei das Gehäuse einen Anschlag und/oder ein sich innerhalb der spiralförmigen Anordnung befindliches Begrenzungselement (112) aufweist, der einen möglichen Rotationsbereich um die Rotationsachse (R) begrenzt.

9. MR-Lokalspule nach einem der Ansprüche 6 bis 8,
wobei die MR-Lokalspule (100) eine Starr-Flex-Leiterplatte (105) umfasst,
wobei das Übertragungselement ein flexibler Teil der Starr-Flex-Leiterplatte (105) ist,
wobei die Starr-Flex-Leiterplatte (105) einen ersten starren Teil umfasst, der mittels einer ersten Verbindung mit dem ersten Gehäuseteil (101) verbunden ist,
wobei die Starr-Flex-Leiterplatte (105) einen zweiten starren Teil umfasst, der mittels einer zweiten Verbindung mit dem zweiten Gehäuseteil verbunden ist.

10. MR-Lokalspule nach Anspruch 9,
wobei die erste Verbindung und/oder die zweite Verbindung lösbar ausgebildet sind.

11. MR-Lokalspule nach einem der Ansprüche 6 bis 10,
wobei das erste Gehäuseteil (101) und/oder das zweite Gehäuseteil (102) einen Spalt (119) umfasst, der senkrecht zur Rotationsachse verläuft,
wobei das Übertragungselement (107) durch den Spalt (119) durchgeführt ist.

12. MR-Lokalspule nach einem der Ansprüche 6 bis 11
wobei das Gehäuse ein Führungselement (118) umfasst,
wobei zumindest der um die Rotationsachse (R) herum spiralförmig angeordnete Teil des Übertragungselements (107) in dem Führungselement (118) angeordnet ist und von diesem geführt wird.

13. MR-Lokalspule nach einem der vorangehenden Ansprüche,
wobei die spiralförmige Anordnung des Übertragungselements (107) um die Rotationsachse (R) herum ein erstes Ende und ein zweites Ende aufweist,
wobei das Übertragungselement (107) am ersten Ende und/oder am zweiten Ende, insbesondere um 90°, abgewinkelt ist.

14. MR-Lokalspule nach Anspruch 13,
wobei das Übertragungselement (107) am ersten Ende und/oder am zweiten Ende um 90° abgewinkelt ist.

15. Magnetresonanzvorrichtung (10) mit zumindest einer MR-Lokalspule (100) nach einem der Ansprüche 1 bis 14.

## Claims

1. MR local coil comprising
- an antenna unit (120) having at least one antenna for receiving and/or transmitting HF signals,
- a connection cable (116) for connecting the MR local coil (100) to a magnetic resonance apparatus (10),
- a flat transmission element (107) for transmitting energy and/or signals between the connection cable (116) and the antenna unit (120),
wherein the connection cable is designed so as to be rotated on a connecting site of the connection cable to the antenna unit about an axis of rotation relative to the antenna unit,
**characterised in that** the transmission element (107) is at least in part arranged about the axis of rotation (R) in a spiral manner, wherein the transmission element describes a coil having at least one half winding about the axis of rotation.

2. MR local coil according to claim 1,
wherein the transmission element (107) comprises a flexible circuit board and/or a flat ribbon cable.

3. MR local coil according to one of the preceding claims, wherein the antenna unit (120) comprises multiple antennas, wherein the transmission element (107) comprises multiple electrical and/or optical conductors, which are arranged adjacent to one another, in order to transmit the signals of the multiple antennas.

4. MR local coil according to one of the preceding claims, wherein the antenna unit (120) is designed as flat.

5. MR local coil according to one of the preceding claims, wherein the antenna unit (120) is designed as flexible.

6. MR local coil according to one of the preceding claims,
wherein the MR local coil (100) comprises a housing having a first housing part (101) and a second housing part (102, 103),
wherein the transmission element (107) is arranged in the housing,
wherein the second housing part (102, 103) can be rotated relative to the first housing part (101) about the axis of rotation (R).

7. MR local coil according to claim 6,
wherein the housing comprises a tongue and groove connection (104),
wherein the second housing part (102, 103) is guided relative to the first housing part (101) by means of the tongue and groove connection (104) in a rotatable manner about the axis of rotation (R).

8. MR local coil according to one of claims 6 or 7,
wherein the housing has a stop and/or a limiting element (112), which is arranged within the spiral-shaped arrangement, and the stop limits a possible rotational range about the axis of rotation (R).

9. MR local coil according to one of claims 6 to 8,
wherein the MR local coil (100) comprises a rigid flexible circuit board (105),
wherein the transmission element is a flexible part of the rigid flexible circuit board (105),
wherein the rigid flexible circuit board (105) comprises a first rigid part that is connected by means of a first connection to the first housing part (101),
wherein the rigid flexible circuit board (105) comprises a second rigid part that is connected by means of a second connection to the second housing part.

10. MR local coil according to claim 9,
wherein the first connection and/or the second connection are designed in a detachable manner.

11. MR local coil according to one of claims 6 to 10,
wherein the first housing part (101) and/or the second housing part (102) comprises a gap (119) that extends perpendicular to the axis of rotation,
wherein the transmission element (107) is guided through the gap (119).

12. MR local coil according to one of claims 6 to 11,
wherein the housing comprises a guiding element (118), wherein at least the part of the transmission element (107), which is arranged in a spiral manner about the axis of rotation (R), is arranged in the guiding element (118) and is guided by said guiding element.

13. MR local coil according to one of the preceding claims,
wherein the spiral-shaped arrangement of the transmission element (107) has a first end and a second end around the axis of rotation (R),
wherein the transmission element (107) is angled at the first end and/or at the second end, in particular by 90°.

14. MR local coil according to claim 13,
wherein the transmission element (107) is angled at the first end and/or the second end by 90°.

15. Magnetic resonance apparatus (10) having at least one MR local coil (100) according to one of claims 1 to 14.

## Revendications

1. Bobine locale RM comprenant :
- une unité (120) d'antenne ayant au moins une antenne de réception et/ou d'émission de signaux HF,
- un câble (116) de connexion pour la connexion de la bobine (100) locale RM à un dispositif (10) de résonnance magnétique,
- un élément (107) de transmission plat pour la transmission d'énergie et/ou de signaux entre le câble (116) de connexion et l'unité (120) d'antenne,
dans laquelle le câble de connexion est constitué pour, en un point de liaison du câble de connexion à l'unité d'antenne, être tourné, par rapport à l'unité d'antenne, autour d'un axe de rotation,
**caractérisée en ce que** l'élément (107) de transmission est disposé en forme de spirale au moins en partie autour de l'axe (R) de rotation, l'élément de transmission décrivant une spirale d'au moins une demi-spire autour de l'axe de rotation.

2. Bobine locale RM suivant la revendication 1,
dans laquelle l'élément (107) de transmission comprend une plaquette à circuit imprimé souple et/ou un câble à bande plate.

3. Bobine locale RM suivant l'une des revendications précédentes,
dans laquelle l'unité (120) d'antenne comprend plusieurs antennes,
dans laquelle l'élément (107) comprend, pour la transmission des signaux des plusieurs antennes, plusieurs conducteurs électriques et/ou optiques disposés les uns à côté des autres.

4. Bobine locale RM suivant l'une des revendications précédentes, dans laquelle l'unité (120) d'antenne est de constitution plate.

5. Bobine locale RM suivant l'une des revendications précédentes,
dans laquelle l'unité (120) d'antenne est de constitution souple.

6. Bobine locale RM suivant l'une des revendications précédentes,
dans laquelle la bobine (100) locale RM comprend un boîtier ayant une première partie (101) de boîtier et une deuxième partie (102, 103) de boîtier,
dans laquelle l'élément (107) de transmission est disposé dans le boîtier,
dans laquelle la deuxième partie (102, 103) de boîtier peut tourner autour de l'axe (R) de rotation par rapport à la première partie (101) de boîtier.

7. Bobine locale RM suivant la revendication 6,
dans laquelle le boîtier comprend un assemblage (104) à tenon et mortaise, dans laquelle la deuxième partie (102, 103) de boîtier peut tourner autour de l'axe (R) de rotation par rapport à la première partie (101) de boîtier en étant guidée au moyen de l'assemblage (104) par tenon et mortaise.

8. Bobine locale RM suivant l'une des revendications 6 ou 7, dans laquelle le boîtier a une butée et/ou un élément (112) de limitation se trouvant à l'intérieur de l'agencement en forme de spirale, qui limite une plage de rotation possible autour de l'axe (R) de rotation.

9. Bobine locale RM suivant l'une des revendications 6 à 8, dans laquelle la bobine (100) locale RM comprend une plaquette (105) à circuit imprimé Starr-Flex,
dans laquelle l'élément de transmission est une partie souple de la plaquette (105) à circuit imprimé Starr-Flex,
dans laquelle la plaquette (105) à circuit imprimé Starr-Flex comprend une première partie rigide, qui est reliée, au moyen d'une première liaison, à la première partie (101) de boîtier,
dans laquelle la plaquette (105) à circuit imprimé Starr-Flex comprend une deuxième partie rigide, qui est reliée à la deuxième partie de boîtier au moyen d'une deuxième liaison.

10. Bobine locale RM suivant la revendication 9,
dans laquelle la première liaison et/ou la deuxième liaison sont constituées de manière détachable.

11. Bobine locale RM suivant l'une des revendications 6 à 10,
dans laquelle la première partie (101) de boîtier et/ou la deuxième partie (102) de boîtier comprend une fente (119), qui s'étend perpendiculairement à l'axe de rotation,
dans laquelle l'élément (107) de transmission passe dans la fente (119).

12. Bobine locale RM suivant l'une des revendications 6 à 11,
dans laquelle le boîtier comprend un élément (118) de guidage,
dans laquelle au moins la partie disposée en forme de spirale autour de l'axe (R) de rotation de l'élément (107) de transmission est disposée dans l'élément (118) de guidage et est guidée par celui-ci.

13. Bobine locale RM suivant l'une des revendications précédentes,
dans laquelle l'agencement en forme de spirale de l'élément (107) de transmission autour de l'axe (R) de rotation a une première extrémité et une deuxième extrémité,
dans laquelle l'élément (107) de transmission est coudé à la première extrémité et/ou à la deuxième extrémité, en particulier de 90°.

14. Bobine locale RM suivant la revendication 13,
dans laquelle l'élément (107) de transmission est coudé à la première extrémité et/ou à la deuxième extrémité de 90°.

15. Dispositif (10) de résonnance magnétique comprenant au moins une bobine (100) locale RM suivant l'une des revendications 1 à 14.
